# EUROPEAN PATENT APPLICATION

(11) **EP 4 691 371 A1**
(43) Date of publication of application: **11.02.2026**
(21) Application number: 24382887.8
(22) Date of filing: 06.08.2024
(51) Int. Cl.: A61B 6/03, A61B 6/50, A01K 1/03

(54) **MOLECULAR IMAGING SYSTEM OF AWAKE ANIMALS**

(71) Applicant: Bruker Española, S.A., 28521 Rivas-Vaciamadrid Madrid (ES)
(72) Inventor: MOLINOS SOLSONA, César, 46013 Valencia (ES); CORRECHER SALVADOR, Carlos, 46013 Valencia (ES)
(74) Representative: Cueto, Sénida

(57) **Abstract**

An imaging system for imaging of an awake and freely moving small animal comprising:
• an animal cage for one or more small animals, transparent to the radiation emitted by the animal, and at least part of the animal cage being transparent to light,
• an enclosure for receiving the animal cage, transparent to the radiation emitted by the animal,
• a detector assembly comprising a plurality of molecular imaging detectors surrounding the enclosure for detecting radiation emitted by the small animal,
• a motion tracking system for optical tracking position and orientation of the animal with optical detectors to synchronize movement and molecular images
• at least a computation unit for processing data.

## Description

### TECHNIQUE SECTOR

The present invention is encompassed within the field of molecular imaging techniques applied to the medical sector.

### STATE OF THE ART

Imaging of the brain in small rodents plays a crucial role in modern biomedical research. It enables the understanding of brain function and disorders. Small rodents such as mice and rats serve as valuable models for studying brain function and neurological disorders. Molecular imaging allows researchers to visualize specific molecules, receptors, and neurotransmitters in the brain. This helps unravel the underlying mechanisms of diseases like Alzheimer's, Parkinson's, and depression. It is also useful in drug development and testing. Small rodents are commonly used in preclinical drug development studies. Molecular imaging enables researchers to track drug distribution, receptor binding, and pharmacological effects in real time. By assessing drug interactions at the molecular level, scientists can optimize treatments and minimize side effects.

One important key feature of molecular imaging techniques like PET is that they enable quantitative assessments. Researchers can quantify receptor availability, neurotransmitter release, and gene expression changes in rodent brains. This information aids in evaluating treatment efficacy and disease progression.

Over the years there have been technological developments at the hardware level with dedicated small-animal PET systems with improved spatial resolution and sensitivity but also important developments in radiochemistry with novel PET imaging probes that allow detailed investigations of brain function. For instance, while dopamine has been extensively studied due to available PET tracers, other neurotransmitter systems (e.g., serotonin, glutamate) are gaining attention. Molecular imaging helps explore these systems and their roles in brain health and disease. The third key technological development has been the availability of animal disease models specially for mice and rats. In addition, most recently techniques like CRISPR/Cas9 and DREADD (Designer Receptors Exclusively Activated by Designer Drugs) enhance our understanding of brain disorders. Imaging complements genetic modifications in animal models, providing insights into human brain conditions.

PET tracers allow researchers to map brain metabolism and perfusion. By monitoring the uptake of these tracers, scientists can gain insights into regional brain activity and blood flow patterns in animals. PET can also be used to monitor neurotransmitter-receptor binding in small laboratory animals. This information is valuable for understanding how specific neurotransmitters interact with receptors and influence brain function. Commercial systems available today invariably require the animal to be taken away from the cage in the vivarium where they live typically as part of a social group and are carried over to the imaging device. In the vast majority of experiments the animal requires to be anesthetized to remain still so imaging can take place.

PET imaging of awake animals allows to study brain function while they are fully conscious. This is crucial because anesthesia, commonly used in imaging studies, can alter brain activity and metabolism.

Nevertheless, rarely is awake animal imaging performed today even in neuro imaging. Only some commercial systems offer accessories to allow awake imaging provided there is immobilization of the head, or the motion is greatly restricted.

Systems allowing to image awake animals have been described in many scientific publications.

Some somewhat awake animal imaging related patents exist, the most relevant being US8170302B1 with a "System and method for generating motion corrected tomographic images" and US8335363B2 describing a "Method for image reconstruction of moving radionuclide source distribution" employing three optical cameras and rotating gantry.

In all these cases, imaging sessions are very time limited (a few hours at most) and involve segregating the animal from their daily living quarters and placing them onto a separated imaging system. The animal is carried over into a location which very often it is not acquainted with, and this also implies the separation from their social group. This generates stress in the animal and interferes in the molecular process to be interrogated as well as affecting its welfare. In addition, although the above systems allow for awake imaging, the subject is not fully able to move about let alone satisfy its vital needs. This imposes a limit to the scope of the study in terms of image acquisition time but also correlation of imaging with natural behavior, social interaction, and stimuli response.

### References

1. "PET imaging of freely moving interacting rats" Miranda et al (last author Verhaeghe) https://www.sciencedirectcom/science/article/pii/S1 053811919301600#bib20
2. "Open-field PET: Simultaneous brain functional imaging and behavioral response measurements in freely moving small animals. Kyme et al., Meikle last author. https://www.sciencedirect.com/science/article/abs/pii/S1053811918321323
3. A Motion Adaptive Animal Chamber for PET Imaging of Freely Moving Animals. Zhou, Meikle. https://ieeexplore.ieee.org/document/6588960
4. Fully conscious PET imaging using motion compensation. Spangler-Bickell et al. Johan Nuyts. Last author. https://link.springer.com/content/pdf/10.1186/s13550-016-0242-3.pdf
5. Real-time 3D motion tracking for small animal brain PET. A Z Kyme and Fulton. https://iopscience.iop.org/article/10.1088/0031-9155/53/10/014
6. RatCAP. https://synchropet.com/ratcap/#:~:text=The%20RatCAP%20is%20a%20small,awake%2 C%20freely%20moving%20small%20rodent.

### DESCRIPTION OF THE INVENTION

The present invention deals with a system that allows molecular imaging of animals in their living quarters over prolonged periods of time without human interference but allows monitoring by humans.

The invention has as an object an imaging system for imaging of an awake and freely moving animal comprising:
- an animal cage for one or more small animals, transparent to the radiation emitted by the animal, and at least part of the animal cage being transparent to light,
- an enclosure for receiving the animal cage, transparent to the radiation emitted by the animal,
- a detector assembly comprising a plurality of molecular imaging detectors surrounding the enclosure for detecting radiation emitted by the small animal,
- a motion tracking system for optical tracking position and orientation of the animal with optical detectors to synchronize movement and molecular images and
- at least a computation unit for processing data.

The animal is intended to be a small animal, such as mice and rats. The invention is not limited to small rodents, but rats and mice are the preferential target species. Other animal models are rabbits, guinea pigs, ferrets, and non-human primates like marmosets. Other non-mammal species are also possible with adaptations (birds, insects, and fish like zebra fish).

"Molecular imaging" is to be understood in the present application as PET, SPECT and preferably is PET.

The radiation can be, for example, PET/SPECT radiation, this is, gamma-radiation.

The cage can be manually inserted or removed from the enclosure. The cage satisfies all the basic vital needs of the animals in it whether used in conjunction with the enclosure or not. During imaging, the cage is locked in a position inside the enclosure, and it does not move.

The cage can have any form and preferably is of parallelepiped shape. According to particular embodiments, the animal cage has a cuboid form.

According to particular embodiments, the animal cage has a cuboid form and the enclosure and the surrounding molecular imaging detectors cover 3 sides of the animal cage in a U-shaped manner. The enclosure provides protection to the detectors.

On the sides of the cage where there are detectors, the detectors need to cover the entire cage, and even have some small margin beyond.

One or several free sides of the cage are reserved to position the optical motion tracking system which also allows for providing light according to the species circadian cycle. According to additional embodiments, the animal cage is transparent to light on at least two neighboring sides allowing the operation of the optical detectors used by the motion tracking system. The transparent sides also allow for visual inspection by humans when the animal cage is not placed in the imaging system.

One side of the cage is reserved to position water and food supply. Food and drink containers can be located outside and connected to inside through orifices. This is necessary to avoid obstructing the view of the motion tracking system. Food can be supplied from one side or the cage ceiling as long as it does not obstruct the motion tracking system. Food and drink are intended to satisfy needs of animal, or they can be part of experiment, for instance administering substances as part of the *in vivo* experiment. The cage can be compatible with air filtration to control air quality. There may be gas ports allowing the administration and scavenging of anesthetics gas, as well as administering of radiotracer gas. Odor stimuli are also possible to be provided.

The cage can be washable and sterilizable. It can be made of glass, plastic or simply be made by metal rods like an ordinary cage. Since the metal rods are thin and sparse structures they can be used to this purpose. A transparent plastic is preferred.

The cage size may vary depending on animal species and must comply with animal welfare regulations. In a preferred embodiment the approximate size is 15 to 20 cm in width, 20 to 30 cm in length and 15 to 20 cm in height.

The molecular imaging detectors are positioned around the cage. The detectors are preferentially stationary with respect to the enclosure although moving detectors can also be used. The detector assembly comprises a minimum of 2 molecular imaging detectors on opposite sides of the animal cage, for example, 2 PET detectors on opposite sides of the cage to enable coincident imaging, particularly PET imaging. Alternatively, 3 or more detectors can be integrated if the size of the enclosure exceeds the size of standard molecular imaging detectors. One possible implementation is to have the molecular imaging detectors located on two opposed walls and the floor of the animal cage and the motion tracking system on the ceiling of the animal cage. In all configurations the imaging system comprises an enclosure which separates the detectors from the animal cage such that the detectors are not contaminated or become filthy with animal excrement.

The data acquisition electronics can be integrated in the detector assembly or be located at a separate location. The design of the imaging system considers costs to make a viable product with widely available technology but also using cutting edge technology like Time of Flight since the electronics is expected to within 5 or 10 years achieve sufficient time resolution to be useful in small animal imaging.

There are many options for tracking objects moving in space so that their location and orientation are detected. The methods available have advanced in the last decades but recently, they have become more accurate and the technology more widely available and therefore simpler to implement.

The precision and complexity of a motion tracking system for accurate spatial location of animals moving freely is relatively high, but there are elements inherent to the application that facilitate the implementation. These elements are the control of lighting conditions, the fact that the motion happens in a confined space and the possibility of using markers or a previous characterization of the subjects to track. Given this, a sufficiently accurate motion tracking system can be implemented using available technology.

The tracking and lighting system is compatible with day-night cycles. Rodents are most active during the dark phase. Infrared illumination and detection can help image tracking in dark conditions.

Multiple cameras can be used to capture footage from different angles and ensure that the entire area is covered depending on the cage size or if there are complex structures or animals which may obstruct the view. Multiple cameras help in providing a more accurate tracking of the sometimes animal's rapid movements and sudden direction changes. In a preferred embodiment optical -based tracking is used and only two cameras are required, their position is fixed.

The motion tracking elements are supported by a structure which is attached to the detector assembly by means of a base plate. The distance between the cameras is always known and this is used as the basis for the depth detection of the object to be tracked.

In the most basic implementation, uniquely identifiable markers are attached to each animal's head. The markers are providing a guiding pattern and are passive by just reflecting light. The markers are either present on stickers on the fur, skin markings or shaved fur patterns. No surgery is involved. If reflective markers are employed, the tracking system is able to detect light coming from them and to accurately determine their positions in 3D space. The two cameras are used to capture the range and define the tracking volume where the markers can move. Specific cameras employed may be sensitive to infrared light which is also reflected by the markers.

In alternative cases no external markers are required. Markers in the form of fiducial radioactive seeds may be applied. This approach has been used for molecular imaging in awake animal. Nevertheless, this method is inherently invasive, and requires handling of radioactive sources. When purely optical markers like reflective patterns are used, these need to be securely positioned which can be difficult and can lead to some discomfort for the animal under investigation. One of the key advantages of the system proposed here is to maximize animal welfare and least interference with the animal life experience.

Therefore, an approach without internal markers is used in the preferred implementation. A software algorithm running on a computer performs the processing of the images captured by the camera and provides the 3D position of the markers as these are being tracked from different camera angles. This allows for the triangulation to produce 3D coordinates and orientation of subjects under study (animal heads).

The motion tracking system and the molecular imaging system both share a common master clock that provides common time stamps to both so that radiation detection events are cross-referenced with known position and orientation of the animal. This enables the image reconstruction as a function of position and orientation that is the basis of freely moving awake animal imaging living in a confined space.

Optionally, one or several optical cameras also offer a live video streamed to the control desk computer and remote devices like cell phones or tablet PCs. Appropriate lighting is provided to ensure that the animal is visible in the video footage as well as to ensure compatibility with the species circadian cycle.

The motion tracking system is not hampered by nest building and other structures can be positioned in a way which does not negatively interfere with the motion tracking system.

The bedding material amount is sufficiently small such that it does not obstruct the vision of the tracking system. Alternatively, transparent nontoxic fabrics are provided for bedding to avoid obscuring view of optical tracking.

The tracking enables the imaging system to discriminate between the subject and its excretions, solid or liquid droppings possibly containing radioactive isotope. Additionally, the signal from droppings can be used to provide information of excretion pathways and biological half-life of the tracer.

A computation unit determines a spatial position and orientation of the small animal from the motion tracking system and thereby overlays images, such as PET/SPECT images at different points in time.

The imaging system allows imaging over long period of times, from minutes to days until cage cleaning is required, which is typically one week for small rodents. Animal welfare is maximized during this time as the system caters for all basic needs of the animal. The animal experience is nearly or equivalent to ordinary cage living. The possibility of using several animals is considered, as rodents are social. By this means isolation for imaging can be avoided. The tracking system can individually identify subjects either through the characteristic external markers or through coarse optical video analysis, such that this information is combined with the molecular imaging to monitor animal behavior and health for disease onset signs based on molecular markers and cognitive decline symptoms based on behavior. This allows to set automatic real time alerts to be sent to the human experiment controllers.

The image can be presented with or without anatomical reference. The anatomical reference can be a previously acquired MRI, CT or an atlas. Other more advanced implementations would allow imaging the entire body even though it is a deformable solid hence the motion tracking and correction becomes more complicated.

The result is a molecular image that varies with time and is presented together with a video showing what the state of the animal was. To aid image interpretation and behavior correlation, a status description is overlaid with the image where there has been an automatic image recognition and categorization of state, for instance: feeding, drinking, sleeping, self-cleaning, social grooming, exploring. In addition, the sum of prolonged acquisition times should also be provided for maximum image quality. The system is also able to provide olfactory, visual, and sound stimuli to correlate with the molecular imaging, but it is not shown here.

Live video streamed to a control station, or mobile devices (cell phone or tablet PC) allows monitoring of the animals condition. This includes alarm function to alert of health condition changes of one or more small animals. The system can include physiological signal monitoring. Temperature and respiration can be monitored via infrared and optical camera respectively. Infrared can help image tracking in dark conditions.

All data are centralized at a control station. It allows for the storage of large amounts of raw data, images etc. Specialized software is used to analyze the video footage captured by the cameras and track the position of the animal over time. The system may be compatible with at least biosafety level 3 which allows to apply it to infectious disease animal models. To be able to offer this feature, the materials of the cage allow for sterilization The cage, according to particular embodiment, is hermetically closed and the atmosphere inside the cage is controlled through air supply and filter system.

The present invention also refers to a method for molecular imaging of an awake and freely moving animal in an imaging system as previously defined, comprising:
- applying one or more markers to the animal to enable optical tracking, for example, the markers are structured stickers, markings on shaved fur and/or shaved patterns in the fur of the small animal,
- capturing radiation emitted by the small animal with the molecular imaging detectors,
- recording position and orientation of the small animal with the motion tracking system,
- correlating radiation data, position and orientation data so as to obtain a molecular image of the small animal.

One or more markers may be applied to the animal to enable optical tracking. The markers may be 2D stickers of distinct shape, size and color arranged in a 3D way onto the animal's body or markings on shaved fur and/or shaved patterns in the fur of the small animal.

The motion tracking system with its detectors and its lighting system is capable to detect these markers and thereby determine a position and orientation of the small animal at any time in the cage. Should the markers be hidden from the motion tracking system because e.g. the small animal is in a corner of the cage or has turned its body such that the markers are not visible to the optical detectors, the computation unit discards signals collected by the molecular imaging detectors.

Once a time series of data from the molecular imaging detectors and optical detectors has been collected, an image of a region of interest of the small animal can be created. The position and orientation of the small animal are calculated for this purpose. Spatial data derived from the signal of the molecular imaging detectors are corrected with the orientation and position data of the optical detectors so that the region of interest of the small animal is correctly overlaid for each part of the time series of data.

The method can also be used to study the behavior of small animals during the day and at night. For this, illumination of the cage is switched from visible light to infrared light which is not visible to small animals. Markers applied to the small animal must be visible under these light conditions and the optical detectors must be sensitive to infrared radiation.

It is advantageous to examine changes in brain activity of the awake and freely moving animal. To focus on this region of interest only position and orientation of the head of the small animal must be registered by the motion tracking system. Therefore markers must be applied only to the head of the small animal.

Excrements of the small animal may be radioactive and may disturb the evaluation of signals of the molecular imaging detectors. To avoid that, their spatial coordinate in the cage is determined by the motion tracking system and or molecular imaging system. As the animal moves around in the cage, the excrements remain fixed. This allows to separate the signals. Should the small animal come too close to its excrements, signals taken during that time period may be discarded by the computation unit. As a side effect, activity from excrements and urine may reveal information on the tracer excretion pathways

It may be interesting to investigate a small animal when it is in a group of small animals. Social interaction may e.g. change brain activity. For that purpose, only one small animal is administered with a radioactive tracer and at least one further small animal without a radioactive tracer is kept in the animal cage to study the impact of social interaction on the molecular images of the first animal. The first animal has markers which of course may be shielded by the other animals. In that case data registered by the computation unit will be discarded.

During imaging, the cage is locked in a position inside the enclosure, and it does not move.

The source of nuclear radiation can be a radioactive tracer.

According to a preferred embodiment of the method, a first small animal is administered with a radioactive tracer and at least one further small animal without a radioactive tracer is kept in the animal cage to study the impact of social interaction on the molecular images of the first animal.

According to particular embodiments the tracking information signals are analyzed for artificial individual animal recognition.

The primary purpose of the invention is neuro applications, behavioral, psychiatric, stimuli response, neuro disease models. In a first implementation only the reconstruction of the brain is provided as a function of time. The brain inside the skull does not deform so it can be assumed to be a rigid solid.

### DESCRIPTION OF THE FIGURES

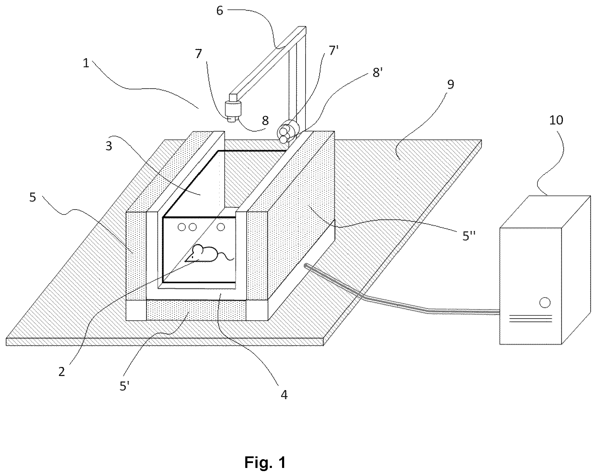

Figure 1 shows a preferred embodiment of a molecular imaging system (1) for imaging of an awake and freely moving small animal (2). An animal cage (3) for one or more small animals is located in an enclosure (4) that prevents direct contact between the animal cage (3) and molecular imaging detectors (5, 5', 5"). The detector assembly surrounds the enclosure at three sides in a U-shaped manner. Since molecular imaging detectors produce artefacts at their edges, the animal cage (3) is smaller than the length and height of the molecular imaging detectors (5, 5', 5"). A motion tracking system (6) comprises two optical detectors (7, 7') and at least one lighting system (8, 8'). The animal cage (3) is transparent to light at least on the sides closest to the optical detectors (7, 7'). It is made of plastic or glass and can be washed and sterilized. The lighting system (8, 8') may emit visible or infrared light for daylight or night cycle observation of the small animal. The optical detectors (7, 7') may be sensitive for visible and/or infrared light to record the motion of the small animal under different conditions. The light sources of the illumination system are arranged in such a way that they do not directly illuminate any of the optical detectors (7, 7'). Otherwise, optical detection of position and orientation of the small animal may suffer from poor contrast. A base plate (9) serves as a holder for the optical detectors (7, 7') and the molecular image detectors (5, 5', 5"). It is important that both systems have a fixed position relative to each other during observation of the small animal. Otherwise blurring of signals registered by the molecular imaging detectors (5, 5', 5") may occur since the spatial reference point provided by the optical detectors (7, 7') may not be stable.

Molecular imaging detectors (5, 5', 5") and optical detectors (7, 7') are connected to a computation unit (10). The computation unit (10) receives data from the molecular imaging detectors (5, 5', 5") and the optical detectors (7, 7'). Moreover, it controls the lighting system (8,8') to adjust intensity and change wavelength range (visible and/or infrared) of the light sources of the lighting system (8, 8'). Markers applied previously to the small animal enable determining position and orientation of the small animal with the signals received by the optical detectors (7, 7').

Since the small animal has been administered with a radioactive tracer before it entered the animal cage (3), the tracers signals can be registered by the molecular imaging detectors (5, 5', 5"). Signals provided by the molecular imaging detectors (5, 5', 5") are processed and correlated with position and orientation of the small animal. By correcting position and orientation of the small animal time series of these data sets can be overlaid to create a higher intensity image of the freely moving small animal.

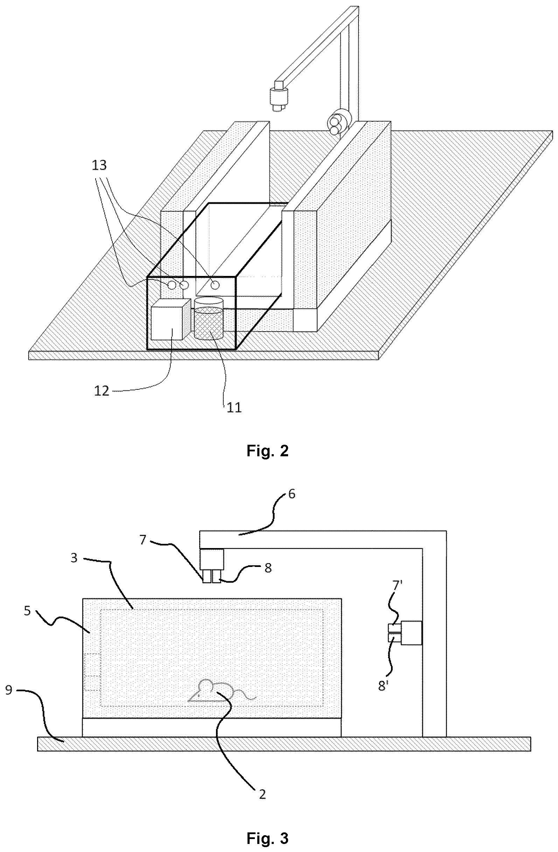

Figure 2 shows cage (3) can be manually inserted or removed from the enclosure (4). The cage has a drink reservoir (11) and a food supply (12) at its front side. Registration of tracer signals emitted by the small animal is not obstructed or shadowed. Providing nutrition allows the small animal to stay within the cage for hours up to some days. Furthermore, activity of the small animal can be observed under different conditions: sleeping, moving, eating and drinking. For long term observation embedding material made of e.g. nontoxic fabrics is supplied at the bottom of the cage (3) to absorb excrements of the small animal. The cage (3) may have gas ports (13) for allowing the administration and scavenging of anesthetics gas, as well as administering of radiotracer gas. Gas ports (13) allow for filtration of the gas circulated through the cage. It must be noted that air circulation must also be controlled when handling radioactive substances, as radioactive tracers must not simply be allowed to enter the ambient air of the cage (3).

Figure 3 shows a side view of the imaging system.

## Claims

1. A molecular imaging system for imaging of an awake and freely moving small animal comprising:
• an animal cage for one or more small animals, transparent to the radiation emitted by the animal, and at least part of the animal cage being transparent to light,
• an enclosure for receiving the animal cage, transparent to the radiation emitted by the animal,
• a detector assembly comprising a plurality of molecular imaging detectors surrounding the enclosure for detecting radiation emitted by the small animal,
• a motion tracking system for optical tracking position and orientation of the animal with optical detectors to synchronize movement and molecular images
• at least a computation unit for processing data.

2. A molecular imaging system according to claim 1, wherein the animal is selected among a mouse, a rat, a guinea pig, a ferret, and non-human primate.

3. A molecular imaging system according to claim 1, wherein the molecular imaging technique is selected among PET and SPECT.

4. An imaging system according to claim 1, wherein, the animal cage is transparent to light on at least two neighboring sides.

5. An imaging system according to claim 1 or 4, wherein the animal cage has a cuboid form.

6. An imaging system according to claim 1, wherein the enclosure and the surrounding molecular imaging detectors cover at least 3 sides of the animal cage in a U-shaped manner.

7. An imaging system according to claim 1, wherein the optical detectors comprise at least two cameras arranged on the at least two sides.

8. An imaging system according to any of claims 1-7, wherein the imaging system comprises a lighting system for illuminating the inside of the animal cage.

9. An imaging system according to claim 8, wherein the lighting system comprises a visible light source and/or an infrared light source and the optical detectors can detect visible and/or infrared light.

10. An imaging system according to any of the previous claims, wherein the molecular imaging detectors are located on two opposed walls of the animal cage and the floor of the animal cage, and the motion tracking system is located on the ceiling of the animal cage.

11. A method for molecular imaging of an awake and freely moving animal in an imaging system as defined in any of the previous claims comprising:
- applying one or more markers to the animal to enable optical tracking, wherein the markers are structured stickers, markings on shaved fur and/or shaved patterns in the fur of the small animal,
- capturing radiation emitted by the small animal with the molecular imaging detectors,
- recording position and orientation of the small animal with the motion tracking system,
- correlating radiation data, position and orientation data so as to obtain a molecular image of the small animal.

12. A method according to claim 11, wherein a lighting system is switched from visible lighting to infrared lighting to simulate a day and night cycle.

13. A method according to any of claims 11-12, wherein markers are applied to the head of a small animal and the brain of the animal is evaluated by the computation unit.

14. A method according to any of claims 11-13, wherein excrements of the small animal are separated from the small animal by the computation unit.

15. A method according to any of claims 11-14, wherein a first small animal is administered with a radioactive tracer and at least one further small animal without a radioactive tracer is kept in the animal cage to study the impact of social interaction on the molecular images of the first animal.
